# EUROPEAN PATENT APPLICATION

(11) **EP 4 317 966 A1**
(43) Date of publication of application: **07.02.2024**
(21) Application number: 21932806.9
(22) Date of filing: 26.03.2021
(51) Int. Cl.: G01N 30/96

(54) **SYSTEM FOR THE ANALYSIS OF BIOMARKERS IN BIOLOGICAL SAMPLES**

(71) Applicant: Thalictrum Global Health, S.L., 46980 Paterna (ES); Fundación Eurecat, 08290 Cerdanyola Del Vallès (ES)
(72) Inventor: GARCÍA VALLS, Ricard, 43893 Altafulla (ES); NOGALSKA, Adriana, 43007 Tarragona (ES); SHAVEL, Alexei, 43007 Tarragona (ES); PELÁEZ CATALÁ, Pedro David, 46182 Paterna (ES)
(74) Representative: Polo Montañes, Carlos
(86) International application number: PCT/ES2021/070213
(87) International publication number: WO 2022/200643

(57) **Abstract**

The invention consists of a sensor device (8) that enables molecules to be broken down into electrons and protons by means of a redox reaction and, according to the specificity given to the sensor, the device generates an electric charge specific to each molecule to be detected. The sensor is complemented with measuring equipment (20) or reading and interpretation equipment for measuring, reading or interpreting the values of the sensor, thus allowing an accurate account of the amount of energy that the sensor is returning to be kept. The invention generates a digital fingerprint of the protein and additionally allows the qualification and quantification thereof.

## Description

### FIELD OF THE ART

The present invention relates to a system for the analysis and digitalization of biomarkers in biological samples through conversion into a scale of electrical values which are generated by molecules being broken down into protons and electrons by means of a chemical process.

According to the specificity given to the sensor, this technology allows the electrical value generated by the biomarkers to be represented in digital format. This technology can thus be used for rapid diagnosis in the treatment and prevention of diseases.

The object of the invention is to provide portable, quick, reliable, and cost-effective analytical means of mass application which allow replacing current laboratory analysis techniques.

### BACKGROUND OF THE INVENTION

Currently, in the field of practical application of the invention, there are technologies in different stages of development and with the possibility of being introduced into the market that can provide improvements over the weak points of the PCR technique for genomic analysis.

Biosensor devices stand out due to their great potential. Conceptually, they are similar to glucose sensors which are used globally today, with there being several configurations in different stages of development (at the laboratory level or more advanced prototypes that are already on the market) which can contribute to the diagnosis of diseases and in health emergencies such as the current one.

Biosensors are integrated autonomous devices made up of a sensor chip in contact with selective biological molecules (antigens, antibodies, or DNA probes specific for that substance, molecule, or pathogen to be detected are placed on the surface of the sensor, for example).

The capture of target molecule causes physicochemical changes which are detected by the transducer and immediately processed into a quantifiable numerical value.

The main advantages of biosensor devices are their decentralized use (outside analysis laboratories) in either primary care or tertiary care centers in emergency rooms of a hospital), their potential of being handled by unskilled staff, their high sensitivity, relatively short analysis time (several minutes), and their ability to offer quantitative values, if necessary.

Furthermore, according to the type of biosensor, it can offer real time measurements without having to amplify signals or use labeling (for example, fluorescent or colorimetric labeling).

Finally, it is important to point out their high versatility in terms of the variety of analyses that can be performed. Sensor chips with different immobilized receptors, i.e., DNA probes, proteins, antibodies, etc., may be obtained.

In the case of genomic detection, optical biosensors (based on microelectronic technology) can possibly be used for the direct detection of viral RNA fragments, without having to carry out amplification cycles which delay the result.

This technology offers extremely high levels of sensitivity, which allows amplification to be avoided, reducing analysis time to a few minutes, minimizing the influence of contamination (since amplification is also dispensed with, increasing reproducibility), and offering quantitative values, therefore correlating the result with a higher or lower amount of genomic material, and therefore of viral load.

However, this option is still not on the market. Alternatives such as the use of fluorescent microarrays can also be useful due to their ability to detect various sequences simultaneously, or alternatively to detect a large number of samples simultaneously. However, this well-established technology requires centralized laboratory instrumentation and skilled staff to interpret same, its sensitivity can be limited, and it would not offer quantitative information, so it is not a more favorable option compared to PCR.

### DESCRIPTION OF THE INVENTION

The proposed system for the analysis of biomarkers in biological samples solves the aforementioned problems in a fully satisfactory manner.

To that end and more specifically, there are two fundamental elements in the system of the invention:
- A sensor device that enables molecules to be broken down into electrons and protons by means of a redox reaction and, according to the specificity given to the sensor, the device generates an electric charge specific to each molecule to be detected.
- Measuring equipment or reading and interpretation equipment for measuring, reading or interpreting the values of the sensor

The purpose of the system is to identify the presence or absence of desired biomarkers by means of the specificity of the sensor in a biological sample in an unambiguous manner and in real time regardless of whether or not external physiological signals or symptoms are absent.

In terms of the sensor, there are three layers in the sensor, a first layer is an FR4 material support sheet with two faces, an upper face that is oriented outwards and determines a reading electrode of the cathode, in which there is established a sample deposition and electrical connection cavity materialized as a through perforation or THT (Through-Hole Technology) going from the upper surface to the lower surface, which lower surface determines an electron collector cathode sheet electrode.

The second layer, or intermediate layer, is materialized as an MEA polymer membrane with an upper layer with the deposition of nanoparticles according to the specificity of the sensor, and a lower active carbon layer, in which the upper layer with the deposition of nanoparticles is in contact and permanent pressure with the electron collector cathode sheet, with connection channels being established between both.

In terms of the third layer, it is materialized as a FR4 material support sheet, on the upper face of which there is established a proton collector cathode sheet or electrode in contact and under pressure with the active carbon layer. The sheet has through perforations or THT (Through-Hole Technology) used as means for extracting gases or fluids generated in the reaction, being finished in the lower portion thereof with a reading electrode of the anode.

The sensor is complemented with a biological sample collection device for collecting biological samples in the form of saliva or another dilution insertable into a sample deposition opening in the cathode.

Based on this structuring, the anode and the cathode are connected to respective system or measuring equipment reading lines.

In turn, the measuring equipment is made up of a central logic and control unit, a storage memory block, an LTE/NB/WIFI wireless connectivity unit block, an integrated power or power source management block, a screen for real time representation and information, RGB LEDs which indicate the state and a high-precision block, coulomb, volt peak and ampere peak measuring unit, thus allowing an accurate account of the amount of energy that the sensor is returning to be kept, and it generates a digital fingerprint of the protein and additionally allows the qualification and quantification thereof.

### DESCRIPTION OF THE DRAWINGS

To complement the description that will be made below and in order to help better understand the features of the invention according to a preferred practical embodiment thereof, a set of drawings is attached as an integral part of said description in which the following is depicted in an illustrative and non-limiting manner:
Figure 1 shows an exploded detail of the structure of the sensor in a system for the analysis of biomarkers in biological samples performed according to the object of the present invention.
Figures 2 and 3 shows respective views of the sensor on which a biological sample collection device for collecting biological samples in the form of saliva or another dilution is applied.
Figure 4 shows a view similar to that of Figure 2, but in which the measuring equipment reading lines can be seen depicted on the sensor.
Figure 5 shows a block diagram of the internal structure of the measuring equipment.
Figure 6 shows an operation diagram of the system.
Figure 7 shows a depiction of the classification results obtained by means of the system of the invention.
Finally, Figure 8 shows a schematic diagram of the connection of the system with a computer database.

### PREFERRED EMBODIMENT OF THE INVENTION

In view of the mentioned figures, it can be observed how the system of the invention is made up of a sensor device and measuring equipment or reading and interpretation equipment for measuring, reading or interpreting the values of the sensor.

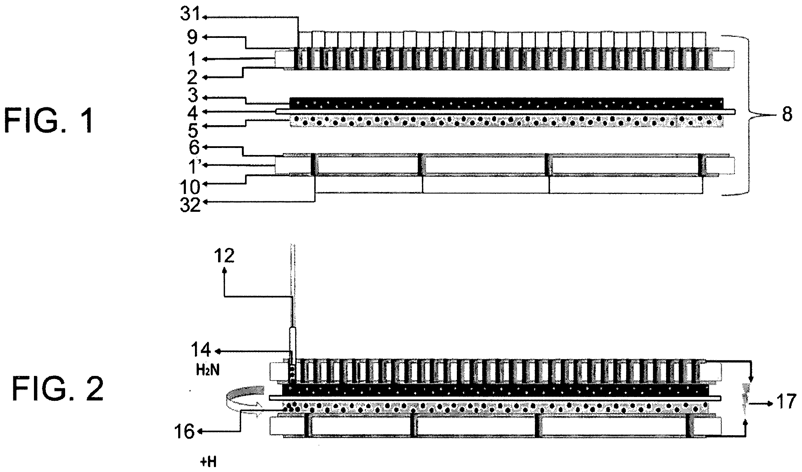

The structure of the sensor (8) is made up of a construction of 3 layers which are differentiated as in Figure 1.

The first layer is an FR4 material support sheet (1) with two faces, an upper face that is oriented outwards and determines the reading electrode of the cathode (9), there is arranged on this surface a sample deposition and electrical connection cavity (31) which is a through perforation or THT (Through-Hole Technology) going from the upper surface to the lower surface, determining an electron collector cathode sheet electrode (2).

The intermediate layer is a MEA polymer membrane (4) with an upper layer, i.e., the upper MEA surface with the deposition of nanoparticles (3) (recipe for specificity), and a lower layer, i.e., an active carbon surface (5). The upper layer (3) of the MEA is in contact and permanent pressure with the lower face of the layer first (2) in order to achieve contact and electrical continuity between the two faces and to thereby enable the transfer of electrons through the electrodes and the connection channels (31) from surface (3) to surface (2), carrying the electrons to the outer surface (9) and allowing generated negative charge to be obtained through the cathode.

The layer third is an FR4 material support sheet (1') with two faces, an upper face that determines the proton collector cathode sheet electrode (6) which is in contact and under pressure with the active carbon surface (5), furthermore it has through perforations or THT (Through-Hole Technology) used as means for extracting gases or fluids generated in the reaction. Another lower outer face is the reading electrode of the anode (10).

Connecting (6) and (5) by pressure allows protons to be transferred through the electrodes, the sample deposition and electrical connection cavity (31) from surface (5) to surface (6), carrying the protons to the outer surface (10) and allowing generated positive charge to be obtained through the anode. In this way, a signal (17) which would be the potential difference between (10) and (9) is obtained.

According to Figure 2, once constructed and depending on the specificity of the upper MEA surface with the deposition of nanoparticles (recipe for specificity) (3) and in some cases also on the active carbon surface (5), the sensor (8) allows the deposition of a biological sample (14) in the form of saliva or another dilution by means of collection with a biological sample collection device (12) (swab or another device).

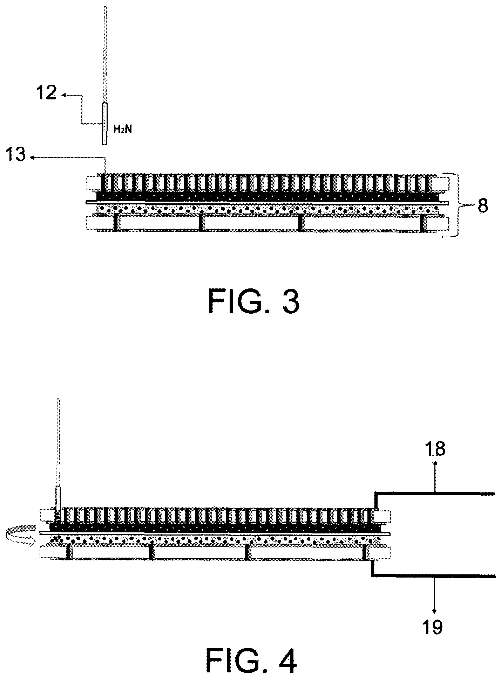

The sample (14) is placed in a sample deposition opening in the cathode (13) for it to be broken down, promoting the emission of electrons and protons (16) as the sample comes into contact with the upper MEA surface with the deposition of nanoparticles (recipe for specificity) (3) and only when the presence or trace of the desired molecule is detected in the sample, and when it is found to be present, an attraction will be generated by the nanoparticles on surface (3) which will break down the molecule into electrons and protons (16), allowing the protons (16), given the configuration of the MEA polymer membrane (4), to be transferred from the upper MEA surface with the deposition of nanoparticles (recipe for specificity) (3) to the active carbon surface (5), going through the MEA polymer membrane (4). Once on the active carbon surface (5), collection of the protons (16) by the proton collector cathode sheet electrode (6) is promoted, transferring those protons through the electrodes located on the surface (10) where the physical connection of the anode measuring system reading line (19) is located, and in the case of electrons to the surface of the reading electrode of the cathode (9), where the physical connection of the cathode measuring system reading line (18) is located, as shown in Figure 4.

The generated electrons and protons (16) will be collected by measuring system reading lines (18) and (19), these lines are connected to signal processing equipment referred to as measuring equipment (20).

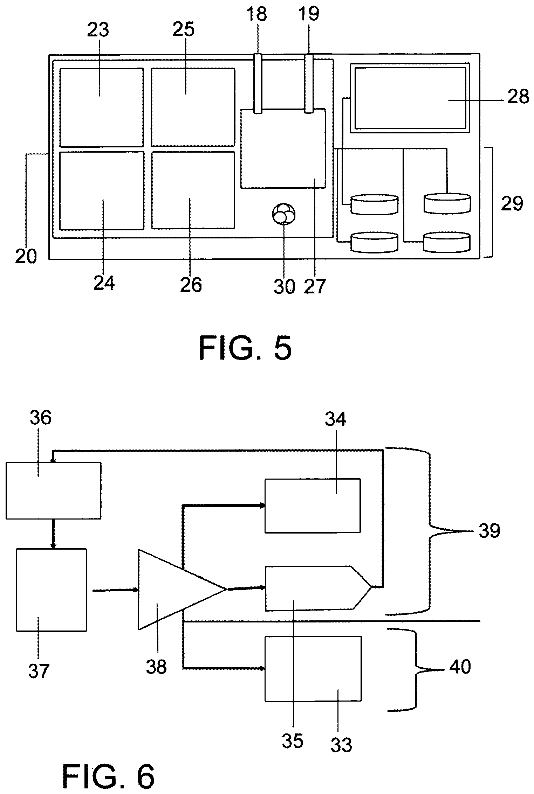

According to Figure 5, the measuring equipment is characterized by different blocks, these electronic blocks being those which will allow collecting, storing and sending data for the control and management of the sensor (8).

The measuring equipment (20) is made up of a central logic and control unit (23) which controls and manages all parts of the device, at the level of each of the blocks making up the measuring equipment (20) itself and has all the configuration parameters for use thereof.

The measuring equipment (20) has therein a storage memory block (24) in which temporary history and information about device configuration are stored, an LTE/NB/WIFI wireless connectivity unit block (25) which allows autonomous connection to an API (application programming interface) cloud system for collecting and storing RAW (unprocessed) data (21). This system as a whole is kept active as a result of the integrated power or power source management block (26), all these blocks are those that allow information use and management, as well as the operation of the measuring equipment (20); in addition to these blocks the measuring equipment (20) has a user interface shown locally in the measuring equipment (20) by means of the screen thereof for real time representation and information (28), the states of the device are represented by RGB LEDs (30) which, depending on the color shown, are understood to mean one state of the device or another, such as the state of being available or not available for sample reading. As an important block in the measuring device (20), there is a high-precision block, a coulomb, volt peak and ampere peak measuring unit (27), this block will be responsible for performing high-precision reading and counting of the electrons and protons of lines (18) and (19), counting the amount circulating through the circuit and the direction thereof, allowing an accurate account of the amount of energy that the sensor (8) is returning to be kept and generating a digital fingerprint of the protein and additionally allows the qualification and quantification thereof.

According to Figure 6, the reading result of the sensor (8) will first be interpreted by the measuring equipment (20) which will show on its screen for real time representation and information (28) the quick result of the datum, qualifying as positive, negative, or state of uncertainty.

The sample obtaining and analysis process (36) will be defined according to the objective of the analysis, but the information flow is defined when the measuring equipment (20) performs the whole sample measurement process (37), generating datum which is automatically analyzed in a fraction of seconds by the measurement reading processing system or algorithm, in turn generating a measurement grading in milliseconds.

The grading results encompass 3 states, the negative measurement result state (33), in which it is determined that if a standby state of the sensor (8), A1-standby state with a value X_{A1} (42), is taken as reference, and the value obtained in the sample obtaining and analysis process (36) is equal to or less than X_{A1} (42), a negative measurement result (33) will be obtained given that no electrical modifications have been received in the sensor, if this happens, it means that there is no trace of the desired molecule. Therefore, the value X_{A1} (42) is equal to 0=negative.

Negative = No trace whatsoever is found.

Another state is the positive sample result (34) in which it is determined that with the sensor (8) in the A1-standby state with a value X_{A1} (42), the sample obtaining and analysis process (36) gives a higher A2-excitation value with a value Y_{A2} (44), so Y_{A2} (44) > X_{A1} (42). In this case, the logic determines whether the value Y_{A2} (44) is within the threshold defined in the algorithm (38) to be considered positive, i.e., that the fingerprint is obtained entirely, and it can be determined that the desired molecule certainly present in the sample above certain values, so Y_{A2} (44) is equal to 1=positive.

Positive = considerable traces above the defined threshold of the molecule for which the sensor has been specified are found.

The last state contemplated is the non-conclusive state (35) or state of uncertainty, in which it is determined that, with the sensor in being the A1-standby state with a value X_{A1} (42), the sample obtaining and analysis process (36) gives a value A--uncertainty with a value Z_{A}~ (43) that is between the maximum accepted value of X_{A1} (42) and the lowest value of YA2 (44). Therefore, Z_{A}~ (43) > X_{A1} (42) and at the same time Z_{A}~ (43) < Y_{A2} (44).

Non-negative = something that excites the sensor is found, but is not conclusive, so it can be determined to retest with a new sample acquisition or refer to another more determinative test.

With respect to the non-conclusive state (35), it is always encompassed in the middle part of the scale of accepted values, ZA~ (43) can never be greater than Y_{A2} (44), because the specificity characteristics of the sensor will not allow a reaction greater than that determined in the specificity, and in the case where this occurs, it would be within the positive categorization (34). This information and the results of the first grading will be graded and shown to the user on the screen for real time representation and information (28) of the measuring equipment (20).

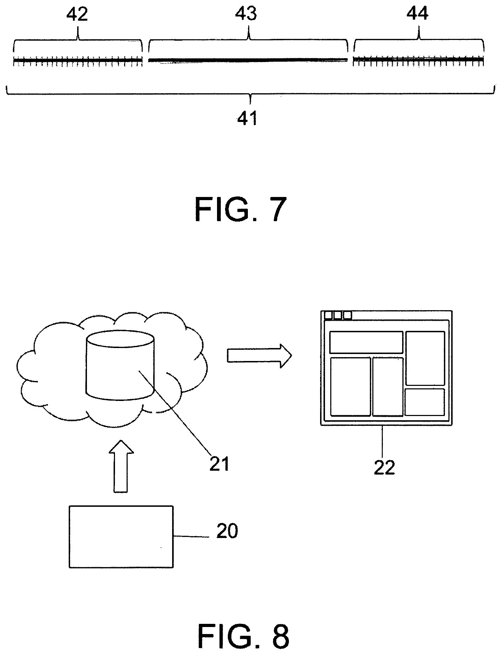

Once information generated by the sensor is available, the measuring equipment (20) sends the structured information to an API cloud system (21) which allows ordering and structuring same in a computer database, as shown in Figure 8.

This database then allows a much more exhaustive sample analysis process to be performed, allowing the generation of a reading quantification, which in reference to a value scale determined by the specificity of the sensor and the previous studies thereof, allows the charge of a molecule in a sample to be quantified, thereby generating quantification.

All the datum generated from each sample is collected and recorded, subsequently allowing an analysis of the collected raw datum in all the variants thereof, potential difference, electric charge, etc.

## Claims

1. A system for the analysis of biomarkers in biological samples, **characterized in that** it is made up of a sensor device (8) provided with means for breaking down molecules into electrons and protons by means of a redox reaction and, according to the specificity given to the sensor (8), the device generates an electric charge specific to each molecule to be detected, with the sensor (8) being connected to measuring equipment (20) or reading and interpretation equipment for measuring, reading or interpreting the values of the sensor (8) provided with means for counting the electrons and protons of lines (18) and (19) that connect the equipment to the sensor (8), as well as the direction thereof, the measuring equipment (20) including means for generating a qualifiable and quantifiable digital fingerprint of the protein being analyzed.

2. The system for the analysis of biomarkers in biological samples according to claim 1, **characterized in that** there are three layers in the sensor (8):
• a first layer is an FR4 material support sheet (1) with two faces, an upper face that is oriented outwards and determines a reading electrode of the cathode (9), in which there is established a sample deposition and electrical connection cavity (31) materialized as a through perforation or THT (Through-Hole Technology) going from the upper surface to the lower surface, which lower surface determines an electron collector cathode sheet electrode (2),
• an intermediate layer materialized as an MEA polymer membrane (4) with an upper layer with the deposition of nanoparticles (3) according to the specificity of the sensor, and a lower active carbon layer (5), in which the upper layer with the deposition of nanoparticles (3) is in contact and permanent pressure with the electron collector cathode sheet (2), with connection channels (31) being established between both,
• a third layer materialized as an FR4 material support sheet (1') on the upper face of which there is established a proton collector cathode sheet or electrode (6) in contact and under pressure with the active carbon layer (5), which sheet has through perforations or THT (Through-Hole Technology) used as means for extracting gases or fluids generated in the reaction, being finished in the lower portion thereof with a reading electrode of the anode (10),
with the anode and the cathode being connected to respective measuring system reading lines (18) and (19).

3. The system for the analysis of biomarkers in biological samples according to claims 1 and 2, **characterized in that** the sensor (8) is complemented with a biological sample collection device (12) for collecting biological samples in the form of saliva or another dilution insertable into a sample deposition opening in the cathode (13).

4. The system for the analysis of biomarkers in biological samples according to claim 1, **characterized in that** the measuring equipment (20) is made up of a central logic and control unit (23), a storage memory block (24), an LTE/NB/WIFI wireless connectivity unit block (25), an integrated power or power source management block (26), a screen for real time representation and information (28), RGB LEDs (30) which indicate the state and a high-precision block, coulomb, volt peak and ampere peak measuring unit (27).
